# EUROPEAN PATENT APPLICATION

(11) **EP 2 082 761 A1**
(43) Date of publication of application: **29.07.2009**
(21) Application number: 08001295.8
(22) Date of filing: 24.01.2008
(51) Int. Cl.: A61M 15/00

(54) **Inhaler**

(71) Applicant: Boehringer Ingelheim International GmbH, 55216 Ingelheim am Rhein (DE)
(72) Inventor: Thoemmes, Ralf, 55216 Ingelheim am Rhein (DE); Frentzel-Beyme, Jessica, 55216 Ingelheim am Rhein (DE)
(74) Representative: Gesthuysen, von Rohr & Eggert

(57) **Abstract**

An inhaler (1) for delivery of a powder-form inhalation formulation from a blister strip (2) with a plurality of blister pockets (3) is proposed. Numbers (18) are arranged between the blister pockets, and the inhaler comprises a display device (17) for displaying the numbers.

## Description

The present invention relates to an inhaler according to the preamble of claim 1 and to a blister strip according to the preamble of claim 8.

The present invention relates to an inhaler for delivery of a powder-form inhalation formulation from a blister strip with a plurality of blister pockets (also called blisters) containing the inhalation formulation in doses.

GB 2 407 042 A discloses an inhaler with a rolled-up blister strip. The inhaler comprises a manually operated, pivotable actuator which operates a conveyor for stepwise moving the blister strip. The actuator supports a piercer and an associated mouthpiece. By pivoting the actuator, the blister strip and be moved forward and blister pockets of the blister strip can be pierced one after the other. When a patient breathes in an air stream passes through the pierced blister pocket, with the result that the inhalation formulation in the blister pocket mixes with the air and is discharged to the patient.

Object of the present invention is to provide an inhaler and a blister strip with simple and/or compact construction allowing counting and/or displaying other information.

The above object is achieved by an inhaler according to claim 1 or a blister strip according to claim 8. Advantageous embodiments are subject of the subclaims.

According to the present invention, the blister strip comprises marks or numbers between the blister pockets. This allows optimized counting and/or display of numbers or any other information, In particular, the numbers or other marks can have an optimized size, which facilitates legibility and, thus, user safety.

Additionally or alternatively, the inhaler comprises a display device for displaying numbers or any other marks between the blister pockets. This allows a very simple and compact construction and/or a high safety.

Further aspects, features, properties and advantages of the present invention are described in the claims and the subsequent description of a preferred embodiment, with reference to the drawing. There are shown in:
- Fig. 1: a schematic sectional view of an inhaler without mouthpiece cover;
- Fig. 2: a schematic sectional representation of the inhaler with closed mouthpiece cover; and
- Fig. 3: a partial, enlarged view of Fig. 2;
- Fig. 4: a side view of the inhaler; and
- Fig. 5: a side view of a blister strip.

In the Figures, the same reference numbers are used for identical or similar parts, even if a repeated description is omitted. In particular identical or corresponding advantages and properties then also result or may be achieved.

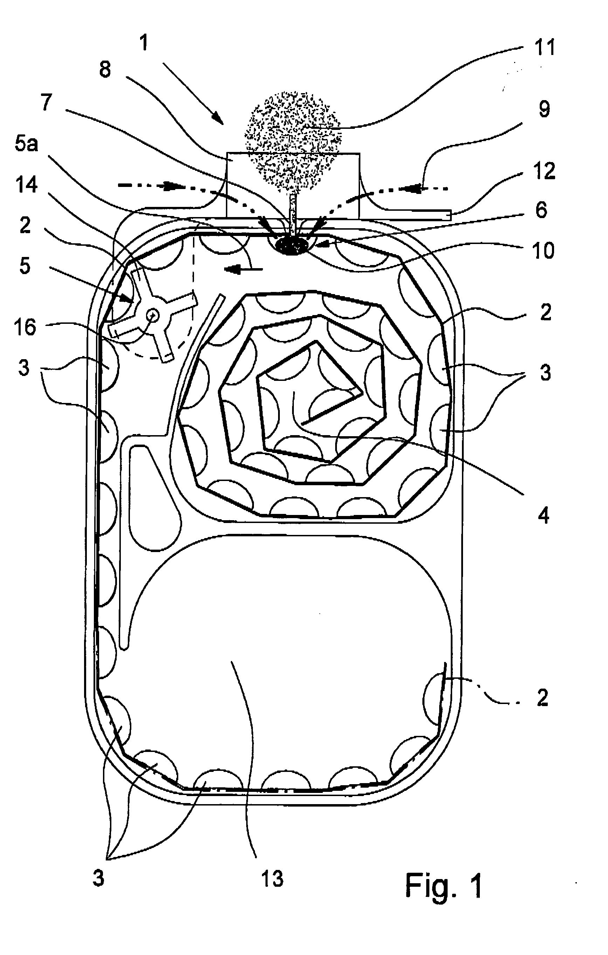

Fig. 1 shows in a schematic sectional representation an inhaler 1.

The inhaler 1 serves to deliver a powdered inhalation formulation from a band-shaped blister strip 2. The blister strip 2 is finite, not forming an endless or closed loop. It has a large number of blister pockets 3 respectively containing directly a dose of the loose inhalation formulation. Thus, the formulation is pre-metered.

The inhaler 1 has a reservoir 4 for the still unused blister strip 2 with closed (sealed) blister pockets 3. The blister strip 3 is rolled up or wound up in the reservoir 4. In the representation example the reservoir 4 is formed such that the blister strip 2 can be moved outwards or pulled out of the reservoir 4 as easily as possible.

In the representation example the blister strip 2 is directly received in the reservoir 4. However, instead of this a cassette, a container, a drum or suchlike can also be fitted or inserted with the blister strip 2 into the inhaler 1 or the reservoir 4.

The inhaler 1 has a conveyor 5 for stepwise onward movement in direction of arrow 5a by one blister pocket 3 in each case, in order to feed the blister pockets 3 successively to an opening and/or removal position 6 where the respective blister pocket 3 is opened and can be emptied.

The blister pockets 3 can be opened respectively preferably by means of a piercing member 7, which punctures cuts open a lid of the respectively aligned blister pocket 3 in position 6. The piercing member 7 is hollow and in fluid connection with an adjacent mouthpiece 8 of the inhaler 1.

During or for inhalation a patient or user, not represented, places the mouthpiece 8 in his mouth and breathes in. The respectively opened blister pocket 3, into which the piercing member 7 extends, is thereby emptied by sucking in. An air stream 9 of ambient air is sucked in and passed through the opened blister pocket 3 such that the loose powder 10 (forming the inhalation formulation and being schematically shown in Fig. 1 only in the actually opened blister pocket 3 below mouthpiece 8) is dispensed with the sucked-in ambient air as an aerosol cloud 11 via the mouthpiece 8. This situation is schematically represented in Fig. 1.

The inhaler 1 has a preferably manually actuateable, lever-like actuator 12 being pivotally mounted to a housing of the inhaler 1.

The piercing member 7 and the mouthpiece 8 are attached to and supported by the actuator 12.

The actuator 12 is operable (pivotable) to cause the piercing member 7 to puncture the lid of the respectively aligned blister pocket 3 in position 6 below the mouthpiece 8.

When the actuator 12 swivels from the position shown in Fig. 1 (here anticlockwise) to a partially opened position, the piercing member 7 is withdrawn from the last-pierced blister pocket 3.

Then, the blister strip 2 is moved forward one blister pocket 3, so that the next blister pocket 3 is moved in position 6. This will be explained in more detail later.

When the actuator 12 swivels back into the position shown in Fig. 1, i.e. is manually moved back, the next aligned blister pocket 3 of the blister strip 2 is punctured by the piercing member 7 and thereby opened. Then, the next inhalation can take place, i.e. the inhaler 1 is activated.

The inhaler 1 has a receiving space or apparatus 13 to receive or store the used part of the blister strip 2. The receiving space or apparatus 13 is preferably formed such that the used part can be wound up. Fig. 1 shows a situation with essentially filled reservoir 4 and still essentially empty receiving space 13.

The conveyor 5 comprises a conveying wheel 14, which can engage between the blister pockets 3 and thus convey the blister strip 2 in form-locking or form-fit manner. This allows very secure or precise moving or indexing of the blister strip 2 as desired and/or necessary.

The conveyor 5 or its conveying wheel 14 is arranged between the reservoir 4 and the receiving apparatus 13, in particular between the removal position 6 and the receiving apparatus 13, thus after the emptying of the blister pockets 3.

The pivot axis of the actuator 12 is coaxial with the rotation axis of the conveying wheel 14. In particular, the actuator 12 may be supported by an axle of the conveying wheel 14.

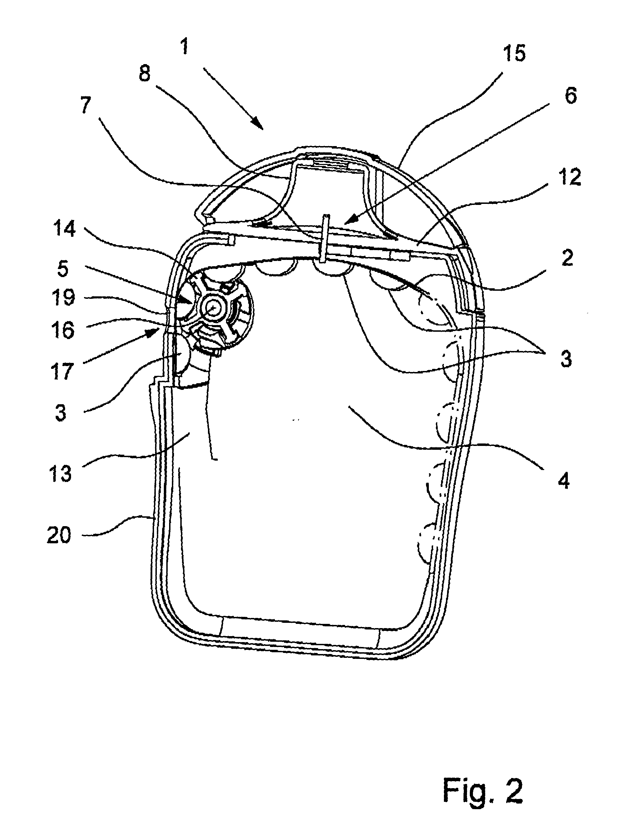

The inhaler 1 comprises a mouthpiece cover 15. The mouthpiece cover 15 is not shown in Fig. 1 which explains the basic principle of the inhaler 1, but in Fig. 2 which shows a more realistic, but still schematic sectional view of the inhaler 1. Fig. 2 shows the inhaler 1 with closed mouthpiece cover 15, wherein the blister strip 2 has been partly omitted for illustration purposes.

The mouthpiece cover 15 is pivotable around a cover axis 16 which is indicated in Fig. 2 and extends perpendicular to the drawing plane in the present representation.

The pivot axis of the actuator 12 extends coaxial to or with the cover axis 16. The rotation axis of the conveying wheel 14 extends coaxial to the cover axis 16 and to the pivot axis of the actuator 12.

The conveyor 5 or its conveying wheel 14 is driven by the mouthpiece cover 15, namely by the pivotal movement of the mouthpiece cover 15. In particular, the blister strip 2 is moved forward when the mouthpiece cover 15 is opened. Preferably, only part of the opening movement of the mouthpiece cover 15 actuates or operates the conveyor 5 or its conveying wheel 14 to move the blister strip 2 forward.

When the mouthpiece cover 15 is opened starting with the completely closed position shown in Fig. 2, in a first phase of the opening movement, for example up to a first angle of about 10, 20 or 30 degrees, the blister strip 2 is not moved due to a respective freewheel (not shown) between the mouthpiece cover 15 and the conveying wheel 14.

First of all, the actuator 12 has to be moved or opened in order to withdraw the piercing member 7 from the previously pierced and usually/already emptied blister pocket 3. This opening movement of the actuator 12 can be performed manually. However, the actuator preferably opens automatically together with the mouthpiece cover 15 or when the mouthpiece cover 15 reaches the first angle.

During the further opening (second phase) of the mouthpiece cover 15, the conveyor 5 or its conveying wheel 14 is actuated to move or index the blister strip 2 by one blister pocket 3 onward to the next blister pocket 3 that shall be emptied. This blister movement happens preferably up to the complete opening of the mouthpiece cover 15.

Only when the mouthpiece cover 15 is opened completely, i.e. reaches its end position, the movement of the blister strip 2 is set or fixed by a respective mechanism (not shown) and/or decoupled from the mouthpiece cover movement to keep the next blister pocket 3 in position 6 for puncturing. However, if the mouthpiece cover 15 is not fully opened and closed again, then the blister strip 2 is moved backward. This facilitates operation of the inhaler 1 and, in particular, prohibits that incomplete or intended operation of the mouthpiece cover 15 results in an undesired movement of the blister strip 2 and eventually in an undesired opening of the next blister pocket 3.

Preferably, a lock (not shown) is provided so that the opened actuator 12 can be closed again only if the mouthpiece cover 15 has been fully opened or has been or has been pivoted back the first angle or if the last-pierced pocket 3 has been moved back into position 6. Thus, the piercing member 7 cannot be pushed against an area of the blister strip 2 without or beside a blister pocket 3.

When the mouthpiece cover 15 has been fully opened and the next blister pocket has been moved in position 6, the actuator 12 can be pivoted back, i.e. closed, in order to pierce the already aligned, still closed blister pocket 3. Then, the inhaler 1 is ready for inhalation, i.e. activated as already described.

After inhalation, the inhaler 1 can be closed by pivoting back the mouthpiece cover 15 into its closed position.

In order to operate the conveyor 5 or its conveying wheel 14 by movement of the mouthpiece cover 15 as described above or in any other suitable manner, the mouthpiece cover 15 is coupled with the conveyor 5, in particular the conveying wheel 14, via the already mentioned freewheel and/or via a suitable transmission, a slipping clutch or any other suitable coupling.

Preferably, the freewheel, transmission, coupling or the like is integrated into or located adjacent to the conveying wheel 14 or a respective axle.

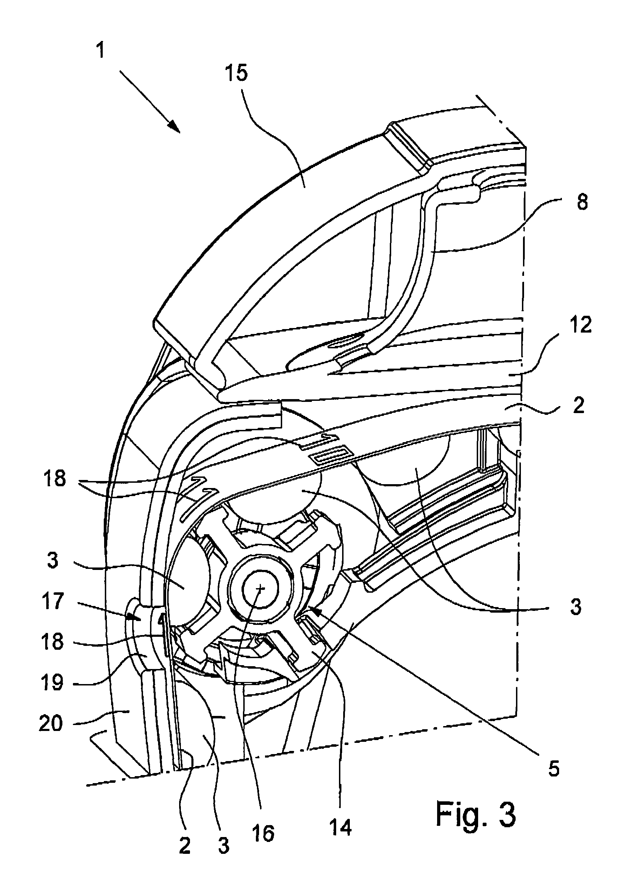

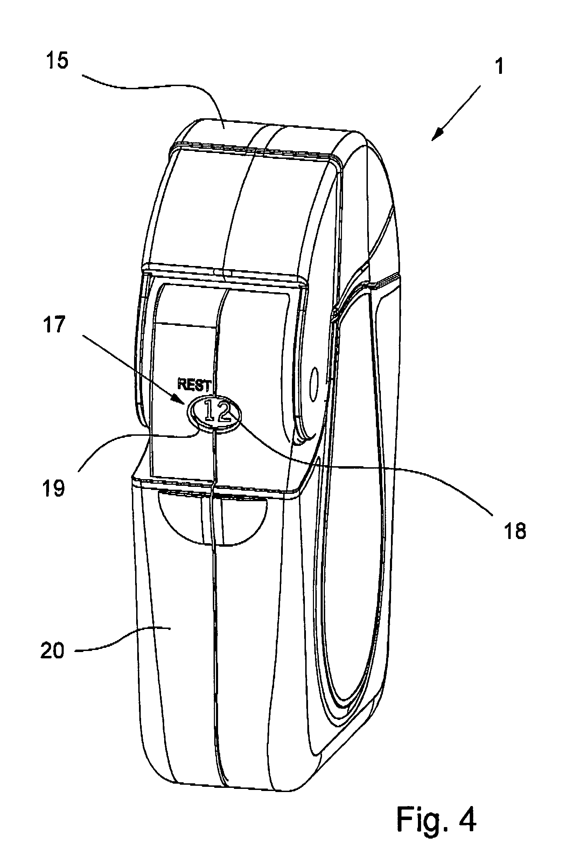

The inhaler 1 preferably comprises a display device 17. In particular, the display device 17 allows to display the total number of already used or of still useable blister pockets 3 and, thus, the respective number of doses of formulation or to display any other information as shown in fig. 3 and the side view according to fig. 4.

The display device 17 preferably comprises a window 19 in the housing 20 of the inhaler 1 or any other suitable component to display the respective number 18 or any other mark. This allows a very simple and reliable construction. In particular any additional components, parts, any transmission or the like are/is not necessary.

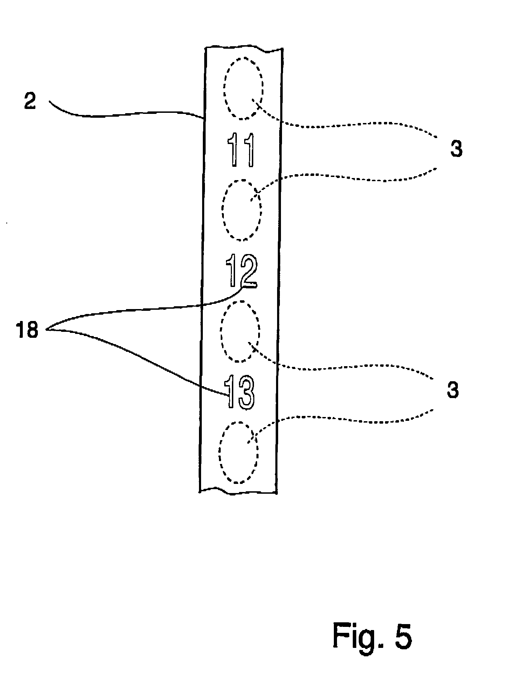

The blister strip 2 comprises numbers 18 or any other marks between the blister pockets 3 as schematically shown in the partial enlarged view of fig. 2 according to fig. 3 and as shown in the enlarged view of a part of the blister strip 2 according to fig. 5.

The marks or numbers 18 are preferably located on the back side of the blister strip 2 and/or on the side of opening the blister strip 2, in particular adjacent to the area where the blister pockets 3 are opened and/or where a foil or the like closes the blister pockets 3.

Therefore, the blister strip 2 can be provided with relatively large numbers 18. In particular the complete width of the blister strip 2 can be used.

The numbers 18 can also slightly extend onto the sealing or covering, here foil, of an adjacent or associated blister pocket 3 as shown in fig. 3. This is considered still as being located (essentially) between the blister pockets 3 according to the present invention.

The numbers 18 may be offset relative to the blister pockets 3 in width direction of the blister strip 2. This is still considered as being located inbetween.

Preferably, the numbers 18 indicate the rest of blister pockets 3 which can still be used, i.e. the total number of the remaining possible uses or inhalations or the total number of already used blister pockets 3.

Preferably, the inhaler 1 is portable, works only mechanically and/or is handheld.

Preferably, the terms "blister strip" and "blister pockets" have to be understood in a very broad sense to cover also other kinds of storage means with receptacles or even bulk storages for the formulation.

### List of reference numbers

- 1: inhaler
- 2: blister strip
- 3: blister pocket
- 4: reservoir
- 5: conveyor
- 5a: onward movement
- 6: opening and/or removal position
- 7: piercing member
- 8: mouthpiece
- 9: air stream
- 10: powder
- 11: aerosol cloud
- 12: actuator
- 13: receiving apparatus
- 14: conveying wheel
- 15: mouthpiece cover
- 16: cover axis
- 17: display device
- 18: number
- 19: window
- 20: housing

## Claims

1. Inhaler (1) for delivery of an inhalation formulation from a preferably band-shaped blister strip (2) with a plurality of blister pockets (3) containing the inhalation formulation in doses, comprising:
preferably a piercing member (7) to puncture an aligned blister pocket (3),
the inhaler (1) being designed such that - preferably by breathing in during inhalation - an air stream (9) of ambient air can be sucked or delivered in order to discharge the respective dose from an opened blister pocket (3) and to deliver it with the ambient air as an aerosol cloud (11),
**characterized in**
**that** the blister strip (2) comprises marks or numbers (18) between the blister pockets (3), and/or
**that** the inhaler (1) comprises a display device (17) for displaying marks or numbers (18) between the blister pockets (3).

2. Inhaler according to claim 1, **characterized in that** the marks or numbers (18) are located at or formed by the flat side of the blister strip (2) and/or the side of individually opening the blister pockets (3).

3. Inhaler according to claim 1 or 2, **characterized in that** the display device (17) comprises a window (19) in a housing (20) of the inhaler (1).

4. Inhaler according to one of the previous claims, **characterized in that** the inhaler (1) is designed such that by breathing in during inhalation an air stream (9) of ambient air can be sucked in order to discharge the respective dose from an opened blister pocket (3) and deliver it with the ambient air as an aerosol cloud (11).

5. Inhaler according to one of the previous claims, **characterized in that** the inhaler (1) comprises a conveyor (5) for preferably stepwise onward movement of the blister strip (2).

6. Inhaler according to one of the previous claims, **characterized in that** the inhalation formulation is powder (10) and/or that the blister pockets (3) contain the inhalation formulation in loose form.

7. Inhaler according to one of the previous claims, **characterized in that** the blister strip (2) does not form a closed loop and is not endless.

8. Blister strip (2) for an inhaler (1) preferably according to any one of the preceding claims, with a plurality of blister pockets (3) containing an inhalation formulation in doses,
**characterized in**
**that** the blister strip (2) comprises marks or numbers (18) between the blister pockets (3).

9. Inhaler according to claim 8, **characterized in that** the marks or numbers (18) are located at or formed by the flat side of the blister strip (2) and/or the side of a foil cover of the blister pockets (3).
